# EUROPEAN PATENT APPLICATION

(11) **EP 3 502 695 A1**
(43) Date of publication of application: **26.06.2019**
(21) Application number: 18151142.9
(22) Date of filing: 11.01.2018
(51) Int. Cl.: G01N 33/543, C12M 3/00, H04L 29/08

(54) **A METHOD AND A SYSTEM FOR ANALYSIS OF CARDIOMYOCYTE FUNCTION**

(30) Priority: 22.12.2017 EP 17210441
(71) Applicant: IMEC vzw, 3001 Leuven (BE)
(72) Inventor: BRAEKEN, Dries, 3001 Leuven (BE); PAUWELYN, Thomas, 3001 Leuven (BE); LAGAE, Liesbet, 3001 Leuven (BE)
(74) Representative: AWA Sweden AB

(57) **Abstract**

A method for analysis of cardiomyocyte function comprises: receiving (302) in a measurement position a substrate (110) carrying cardiomyocytes on a cell culturing surface; recording (304) electrical signals from the cardiomyocytes; simultaneously with said recording, acquiring (306) a sequence of images of the cardiomyocytes on the cell culturing surface based on detecting light wavefront information of reflected light; determining (308) an electrical representation relating to an intracellular and/or extracellular action potential, and/or an impedimetric electrical image of a cardiomyocyte based on the recorded electrical signals; and determining (310) a contractile representation relating to cellular deformation during contractility action of a group of cardiomyocytes based on the acquired sequence of images, wherein the electrical representation and the contractile representation apply to a common period of time.

## Description

### Technical field

The present inventive concept relates to a method and a system for analysis of cardiomyocyte function. In particular, the present inventive concept relates to an analysis which may be utilized for predicting drug adverse effects on cardiac cells.

### Background

In treatment of various different illnesses, drugs are administered to patients. The administered drug may have an impact on cardiac function of the patient. In particular, drugs used in cancer treatments may neqatively impact cardiac function of the patient. In such respect, study of cardiotoxicity of drugs is important. In particular, it may be interesting to study general cardiotoxicity of drugs, but it may also be of particular interest to predict any adverse effects of a drug to a particular patient before deciding on which drug to be administered to the particular patient.

However, a predictive value of current techniques for cardiotoxicity assessment is low. Existing methods lack either resolution, and/or are invasive, and/or are not label-free. It is of obvious importance that the technique is non-invasive to the cells under study, which may allow long term measurements (days to weeks) of effects on the cells. An invasive method would only allow making one measurement, as the measurement would constitute an end point to analysis of the cell.

In US 2016/0017268, devices and method comprising microelectrode arrays for the differentiation, maturation and functional analysis of electroconductive cells are disclosed. The microelectrode present on the arrays can be used to stimulate and record from cells cultured on the substrate.

However, information of functionality of cardiac cells and, hence, cardiotoxicity assessment of drugs, would be improved by information of electrical signals in the cardiac cells being combined with information of contractile movements of the cardiac cells. Therefore, there is a need of improving analysis of cardiomyocyte function and, in particular, for combining electrical and contractile information.

### Summary

An objective of the present inventive concept is to improve analysis of cardiomyocyte function enabling simultaneous analysis of electrical and contractile functionality of the cardiomyocytes. A particular objective of the present inventive concept is to enable improved cardiotoxicity assessment of drugs.

These and other objectives of the invention are at least partly met by the invention as defined in the independent claims. Preferred embodiments are set out in the dependent claims.

According to a first aspect, there is provided a method for analysis of cardiomyocyte function, said method comprising: receiving in a measurement position a substrate carrying cardiomyocytes on a cell culturing surface, wherein the substrate comprises a microelectrode array in contact with the cardiomyocytes; recording electrical signals from the cardiomyocytes, said electrical signals being acquired by the microelectrode array; simultaneously with said recording, acquiring a sequence of images of the cardiomyocytes on the cell culturing surface, wherein each image in the sequence of images is acquired by detecting light wavefront information of reflected light, the light wavefront information forming an interference pattern between diffracted light from the cardiomyocytes and undiffracted light, and digitally calculating image information based on the detected light wavefront information; determining an electrical representation relating to an intracellular and/or extracellular action potential, and/or an impedimetric electrical image of a cardiomyocyte based on the recorded electrical signals, determining a contractile representation relating to cellular deformation during contractility action of a group of cardiomyocytes based on the acquired sequence of images, wherein the electrical representation and the contractile representation apply to a common period of time.

Thanks to recording of electrical signals by means of the microelectrode array and simultaneously acquiring a sequence of images using detection of light wavefront information of reflected light, it is possible to determine both information of electrical function and information of contractile function on a single cell culture formed on a substrate, i.e. on the same cells.

Thanks to the images being acquired based on reflected light, the microelectrode array, which may be arranged below the cardiomyocytes, will not interfere with the acquiring of images. Thus, the recording of electrical signals and acquiring of images may be performed simultaneously, which implies that the electrical and contractile information may be compared in a common period of time, which facilitates analysis of cardiomyocyte function.

Further, both the electrical representation and the contractile representation may be determined without need of any labelling of cells, which implies that the method is non-invasive and that long-term measurement may be performed. Hence, the method may be used for analyzing development or changes in cardiomyocyte function over a large period of time.

The analysis of cardiomyocyte function may be performed using a compact set-up, since the electrical signals may be recorded and the images may be acquired in a common measurement position.

The cardiomyocytes on the cell culturing surface may be cultured based on stem cells of a patient. This implies that the substrate may carry cardiomyocyte cells of a particular patient, such that the analysis of cardiomyocyte function may be related to the particular patient and may give insight for individual treatment of the particular patient.

It should also be realized that the assessment of cardiomyocyte function may be used for general understanding of cardiomyocyte function and may be used e.g. in development of new drugs for treatment of heart disease.

The use of a microelectrode array in contact with the cardiomyocytes enables access to and determining of electrical activity in and between individual cells. Also, thanks to the microelectrode array, it is possible to acquire information of propagation of an excitation wave.

The determined electrical representation may provide a measure of intracellular action potential and/or extracellular action potential. This implies that the electrical representation may provide information relating to opening and closing of ion channels within and/or between cells which play a central role in activation of processes in and/or between cells.

The determined electrical representation may also or alternatively provide a spectroscopic electrical impedance measurement in the form of an electrical impedimetric image. The electrical impedimetric image may give information about cell viability, attachment and movement.

The acquiring of images using detection of light wavefront information enables imaging of the cardiomyocytes without using any labels. The light wavefront information may be acquired using spatially and/or spectrally coherent light, which may enable determining a diffraction of light based on light interacting with cardiomyocytes based on interference of diffracted light with light that has not been diffracted. This may be referred to as digital holography or lensfree imaging.

The undiffracted light need not be diverted into a different optical path. Rather, the interference pattern may be formed based on diffracted and undiffracted light in a common light beam. This enables a simple optical set-up.

An image of a diffracting object may be acquired by calculating or reconstructing an image of the object based on that the detected light wavefront provides information of diffraction occurring in an object plane.

It should be realized that the contractile representation of a group of cardiomyocytes may be determined relating to a group of cardiomyocytes which does not necessarily include a cardiomyocyte for which the electrical representation is determined. Thus, the electrical representation and the contractile representation may relate to different cells. However, the representations may still be formed based on cardiomyocytes in a common cell culture, which may allow analyzing the electrical representation and the contractile representation as applying to the same cells.

In an embodiment, the group of cardiomyocytes includes the cardiomyocyte for which the electrical representation is determined. This implies that the same particular cell is analyzed in both the electrical representation and the contractile representation.

According to an embodiment, a plurality of electrical representations is determined, wherein the plurality of electrical representations relate to different cardiomyocytes. Further, a plurality of contractile representations is determined, wherein the plurality of contractile representations relate to different groups of cardiomyocytes. This may allow analyzing propagation of an excitation wave among the cardiomyocytes by comparing the plurality of electrical representations and the plurality of contractile representations.

According to an embodiment, the method further comprises administering a drug to the cardiomyocytes and comparing the electrical representation and the contractile representation acquired before administering of the drug with the electrical representation and the contractile representation acquired after administering of the drug.

This implies that the analysis of cardiomyocyte function may be utilized for cardiotoxicity assessment of drugs. Both electrical and contractile information may be obtained by the analysis, which enables the assessment of the drug to take into account both electrical and contractile information.

According to an embodiment, the method further comprises, based on said comparing, determining whether the drug has electrical adverse effects on the cardiomyocytes and determining whether the drug has contractile adverse effects on the cardiomyocytes.

Hence, a cardiotoxicity assessment of a drug using the method may allow determining both any electrical adverse effects and any contractile adverse effects. The determination may be performed on the same individual cell culture and may be performed simultaneously.

Further, the determination may be performed based on a long-term measurement, as the method is non-invasive.

According to an embodiment, the method further comprises sending an electrical signal to one or more cardiomyocytes for pacing the cardiomyocytes.

This implies that the action of the cardiomyocytes may be paced, which may enable controlling of when activity of the cardiomyocytes is to occur and hence providing a control of the analysis. This may be especially useful if it is desired to analyze propagation of an excitation wave. Also, pacing the cardiomyocytes may eliminate spontaneous activation of some cardiomyocytes. Thus, by pacing the cardiomyocytes, in vivo activity of a heart may be better simulated.

Pacing may be implemented by means of providing an electrical signal through the microelectrode array to one or more cardiomyocytes on the substrate. However, pacing may alternatively be implemented by providing an electrical signal through dedicated stimulation electrodes, which may be separate from the microelectrode array for recording of electrical signals.

It should be realized that pacing need not necessarily be provided. Cardiomyocytes may spontaneously activate and activity of a cardiomyocyte spontaneously activated may also trigger propagation of an excitation wave through the cardiomyocytes.

According to an embodiment, the method further comprises synchronizing the recorded electrical signals with the acquired sequence of images.

In particular for the sequence of images, processing of data is needed in order to form the sequence of images. Hence, there may be a time delay between the detection of light wavefront information until an image is determined.

The recorded electrical signals and the acquired sequence of images may be synchronized in post-processing in order to enable comparing electrical and contractile information in a common time reference.

It should be realized that the synchronization may occur at any point in time during processing, e.g. for the recorded electrical signals and the acquired sequence of images before determining the electrical representation and the contractile representation or after the representations have been determined.

According to an embodiment, the images in the acquired sequence of images are divided into a plurality of regions of interest, wherein each region of interest represents a group of cardiomyocytes.

This implies that the images are divided such that the contractile representation may be determined based on changes occurring in a specific portion which is the same portion for each of the images in the sequence.

Further, by having a plurality of regions of interest, analysis of propagation of an excitation wave is enabled by simply comparing contractile information for different regions of interest.

According to an embodiment, determining of a contractile representation comprises, for each image, determining a vector sum representing a displacement field in relation to a common reference, and forming a curve profile representing relative cellular deformation based on a sequence of the determined vector sums.

Thus, a measure of cellular deformation may be acquired from each image as a displacement field in relation to a common reference. The measure may then be used in order to form a curve profile representing relative cellular deformation, such that a progress in cellular deformation may be followed and magnitude of relative cellular deformation may be determined. The curve profile representing relative cellular deformation may thus be further analyzed in order to analyze contractile action of the group of cardiomyocytes.

The common reference may be determined specifically for each region of interest and may be based on a single image obtained of the region of interest, which may be selected in an intelligent manner, such as selecting an image halfway between two beats, whereby the image represents a "relaxed" state of the group of cardiomyocytes. The common reference may alternatively be based on a plurality of images obtained of the region of interest, such as an average image based on a plurality of images in a sequence.

According to an embodiment, the determining of a contractile representation further comprises forming a curve profile representing a rate of relative cellular deformation based on a first derivative of the curve profile representing relative cellular deformation.

Thus, another curve profile may be determined representing a rate of relative cellular deformation. This implies that a speed of change of the relative cellular deformation may be analyzed. The curve profile representing rate of relative cellular deformation may thus be further analyzed in combination with the curve profile representing relative cellular deformation or individually in order to analyze contractile action of the group of cardiomyocytes.

According to an embodiment, the method further comprises determining an average curve profile based on a plurality of beat curve profiles for determining the curve profile representing relative cellular deformation and the curve profile representing the rate of relative cellular deformation.

The average curve profile may be used for extracting measures describing the contractile activity of cardiomyocytes. Using an average profile, irregularities in certain beats may be averaged out and the average curve profile may also be less sensitive to noise than curve profiles of individual beats.

A cardiomyocyte may periodically vary in phases of contraction between a contracted state and a relaxed state for contributing to the action of a beating heart. A beat curve profile should be construed as a time sequence in a curve profile between a first point in time corresponding to a specific phase of contraction and a second point in time corresponding to the next time the specific phase of contraction is assumed. Hence, in a time sequence, a plurality of sequential beat curve profiles may occur and the time sequence may be divided into the plurality of beat curve profiles. Each beat curve profile may be individually analyzed or combined with other beat curve profiles for forming an average curve profile.

According to an embodiment, the method further comprises extracting a beating rate from the curve profile representing relative cellular deformation and the curve profile representing the rate of relative cellular deformation.

The beating rate may be an important characteristic to be used in cardiomyocyte function analysis.

The beating rate may be determined based on determining a duration between specific phases of contraction of the cardiomyocyte in sequential beats, such as peak-to-peak durations. Alternatively, a duration over a plurality of beats may be determined in order to determine an average beating rate. As a further alternative, the beating rate may be determined based on a duration of an average curve profile.

According to an embodiment, the method further comprises extracting a contraction duration based on the curve profile representing relative cellular deformation by determining a period of time for which the curve profile exceeds a threshold related to a peak value of the curve profile.

The duration of contraction may also be an important characteristic to be used in cardiomyocyte function analysis. By comparing the curve profile to a threshold, it may be determined whether the contraction is of a sufficient magnitude, such that even if a normal beating rate is provided, it may be identified if the contractile action is too weak.

According to an embodiment, the method further comprising extracting parameters based on a shape of the curve profile representing relative cellular deformation and the curve profile representing the rate of relative cellular deformation.

It should be realized that the shape of the curve profile may provide information that may be extracted in many different ways in order to compare cardiomyocyte function between different cardiomyocytes (in different samples). By extracting such parameters in a common manner, the shape of the curve profiles for different cardiomyocytes may be compared in a simple way.

The parameters could for instance relate to slopes of upstroke, downstroke or plateau phase in a beat curve profile, or relaxation and contraction rate.

According to an embodiment, the method further comprises determining, for each region of interest, a point in time of a phase of contraction, and determining a propagation velocity of an excitation wave based on difference in the point in time for different regions of interest.

Thus, by determining a point in time when a specific phase of contraction is assumed in each region of interest, a progress of an excitation wave may be followed based on the points in time determined for each region of interest. This implies that propagation of the excitation wave based on contractile information may be followed.

The specific phase of contraction may be in point in a beat, such as a peak in the curve profile corresponding to maximal contraction or a minima corresponding to a relaxed state.

The propagation velocity of the excitation wave may be determined based on a difference in points in time for the phase of contraction for regions of interest which are far apart on the substrate. This implies that an average velocity over a relatively large distance may be determined.

According to an embodiment, the method further comprises extracting parameters based on a curve profile representing an intracellular and/or extracellular action potential, and/or an impedimetric electrical image.

The curve profile may be formed based on the recorded electrical signal and may be directly determined as a curve profile representing variation of the electrical signal recorded from a cardiomyocyte. It should be realized that some pre-processing, such as filtering or smoothing, or other more advanced processing of the recorded electrical signal may be performed in order to determine the curve profile.

It should be realized that the shape of the curve profile may provide information that may be extracted in many different ways in order to compare cardiomyocyte function between different cardiomyocytes (in different samples). By extracting such parameters in a common manner, the shape of the curve profiles for different cardiomyocytes may be compared in a simple way.

According to an embodiment, an action potential duration is determined based on the recorded electrical signal from a cardiomyocyte by determining a period of time for which the recorded electrical signal exceeds a threshold related to a peak value of the recorded electrical signal.

This may be a characteristic of the electrical information of specific interest which may allow comparing the electrical activity of a cardiomyocyte to other cardiomyocytes.

According to a second aspect, there is provided a device for analysis of cardiomyocyte function, said device comprising: readout circuitry for reading out electrical signals from a substrate comprising a microelectrode array in contact with cardiomyocytes on a cell culturing surface of the substrate; an image sensor arranged such that the cell culturing surface of the substrate is facing the image sensor when the substrate is arranged to allow the readout circuitry to read out the electrical signals, the image sensor being configured to, simultaneously with reading out of electrical signals by the readout circuitry, acquiring a sequence of images of the cardiomyocytes on the cell culturing surface, wherein each image in the sequence of images is acquired by detecting light wavefront information of reflected light, the light wavefront information forming an interference pattern between diffracted light from the cardiomyocytes and undiffracted light, and digitally calculating image information based on the detected light wavefront information; a processing unit configured to receive the electrical signals from the readout circuitry and image information from the image sensor, said processing unit being further configured to determine an electrical representation relating to an intracellular action potential of a cardiomyocyte based on the recorded electrical signals, and to determine a contractile representation relating to cellular deformation during contractility action of a group of cardiomyocytes based on the acquired sequence of images, wherein the electrical representation and the contractile representation apply to a common period of time.

Effects and features of this second aspect are largely analogous to those described above in connection with the first aspect. Embodiments mentioned in relation to the first aspect are largely compatible with the second aspect.

The device may provide an analysis equipment for analyzing cardiomyocyte function in relation to both electrical and contractile information for a single cell culture. Using the device, electrical and contractile information for a common period of time may be determined, which facilitates analysis of cardiomyocyte function.

Further, the device may provide a compact set-up, where the readout circuitry for reading out electrical signals and the image sensor for acquiring image information are arranged in such manner as to not interfere with respective measurements, while allowing measurements to be simultaneously performed.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a schematic view of a device for analysis of cardiomyocyte function according to an embodiment.
Fig. 2 is a flowchart of a method utilizing analysis of cardiomyocyte function.
Fig. 3 is a flowchart of a method of analyzing cardiomyocyte function according to an embodiment.
Fig. 4 is a schematic view of an electrical representation and a contractile representation.
Fig. 5 is a schematic view of an electrical representation and a contractile representation before and after administering of a drug.
Fig. 6 is a schematic view of relative cellular deformation and rate of relative cellular deformation in dependence of concentration of drug being administered.

### Detailed description

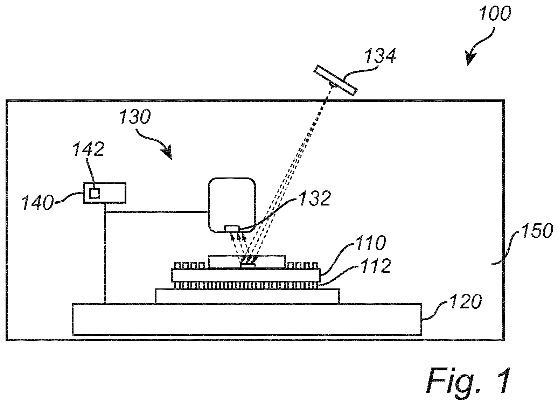

Referring now to Fig. 1, a device 100 for analysis of cardiomyocyte function will be generally described. The device 100 may define a measurement position, in which a measurement object may be placed. Thus, a substrate 110 carrying cardiomyocytes on a cell culturing surface may be placed in the measurement position. The device 100 is configured to determine electrical representation and contractile representation defining electrical and contractile activity of the cardiomyocytes.

The substrate 110 may comprise a microelectrode array 112 in contact with the cardiomyocytes on the cell culturing surface of the substrate. The microelectrode array 112 may thus record electrical signals from the cardiomyocytes.

The microelectrode array 112 may provide a dense arrangement of electrodes such that the electrical signals of a plurality of cardiomyocytes may be recorded. In an embodiment, the microelectrode array 112 may be configured to record electrical signals of each cardiomyocyte on the cell culturing surface.

The microelectrode array 112 may also provide stimulating electrical signals to the cardiomyocytes or at least to some of the cardiomyocytes. Thus, pacing of cardiomyocyte function may be provided during analysis of cardiomyocyte function by the device 100.

The device 100 may comprise a readout circuitry 120 for reading electrical signals from the microelectrode array 112. The readout circuitry 120 may comprise an interface for connecting the microelectrode array 112 to the readout circuitry 120, when the substrate 110 is arranged in the measurement position. The interface may be a single connector or a plurality of connectors for providing transfer of electrical signals between the microelectrode array 112 and the readout circuitry 120.

The device 100 may further comprise an image sensor 130. The image sensor 130 may be arranged in relation to the measurement position, such that the cell culturing surface of the substrate 110 will be facing the image sensor 130 when the substrate 110 is arranged in the measurement position. This implies that the image sensor 130 may be arranged above the measurement position with the readout circuitry 120 being arranged below the measurement position, which may provide a very compact set-up of the device 100. However, it should be realized that other arrangements may be possible. For instance, the readout circuitry 120 may be arranged at a side of the substrate 110, or the readout circuitry 120 may be connected via a cable to the microelectrode array 112 such that the readout circuitry 120 need not be arranged immediately close to the measurement position.

The image sensor 130 may comprise an array 132 of light-detecting elements for detecting an intensity of light incident on the light-detecting elements. The array 132 may for instance be formed as a charge-coupled device (CCD), or an array of complementary metal-oxide-semiconductor (CMOS) light-sensitive elements.

The image sensor 130 may be used for detecting light wavefront information. Hence, an interference pattern may be detected, which may be formed based on interference between light having been diffracted by the cardiomyocytes and undiffracted light, i.e. light not interacting with the cardiomyocytes.

The interference pattern may be based on a single light beam, which partly comprises diffracted light and partly comprises undiffracted light in order to form the interference pattern. The interference pattern comprises information of a shape of objects causing diffraction of light, such that an image of the objects may be determined based on processing of the detected light wavefront information. This may be referred to as holographic imaging or lensfree imaging, since no lens is used for forming an image in an image plane. However, this should not be construed as the optical system necessarily being completely lensfree. The optical system may still comprise one or more lenses for guiding light in the optical system.

The undiffracted light and the diffracted light may be present in a single light beam, forming an in-line set-up of the lensfree imaging. However, other set-ups may be contemplated, such as forming two light beams, an object beam for interaction with the object and a reference beam, wherein the two light beams are combined after the object beam has interacted with the object so as to form the interference pattern.

An image of the cardiomyocytes may be calculated using the detected light wavefront information. The light wavefront information in an image plane of the image sensor 130 may be iterated back to an object plane where the cardiomyocytes are arranged so as to determine an image of the cardiomyocytes. Any suitable algorithm for determining the image may be used, as known to the person skilled in the art, such as a Gerchberg-Saxton algorithm.

The device 100 may include a light source 134 for illuminating the cardiomyocytes on the substrate 110 so as to form the interference pattern. The light source 134 may be a laser source providing spatially and spectrally coherent light. However, the light source 134 may alternatively be a light-emitting diode (LED) combined with a pinhole, such that a light beam output through the pinhole forms spatially coherent light.

The use of spatially coherent light may enable acquiring an interference pattern which allows determining a high quality image of the cardiomyocytes.

The cardiomyocytes may be arranged on a reflective surface, such that reflective lensfree imaging may be provided. Thus, the light source 134 and the image sensor 130 may be arranged on a common side in relation to the substrate 110. This implies that the imaging may be performed without the recording of electrical signals simultaneously with the imaging affecting quality of imaging.

The device 100 may further comprise a processing unit 140. The processing unit 140 may be connected to the readout circuitry 120 and may be configured to receive the electrical signals from the readout circuitry 120. The processing unit 140 may further be connected to the image sensor 130 and may be configured to receive image information from the image sensor 130.

The processing unit 140 may be configured to receive a raw signal as acquired by the readout circuitry 120. However, the readout circuitry 120 may be associated with preprocessing circuitry for preprocessing the electrical signal, such as filtering and/or smoothing of the electrical signal. The preprocessing circuitry may be arranged integrated with the readout circuitry 120 or may be arranged in an intermediate unit, which receives signals from the readout circuitry 120, preprocesses the signals and then forwards the preprocessed signal to the processing unit 140.

The processing unit 140 may be configured to receive the detected light wavefront information from the array 132 of light-detecting elements. The processing unit 140 may thus be configured, e.g. in a processing thread of the processing unit 140, to calculate the image information. However, as an alternative, the detected light wavefront information from the array 132 of light-detecting elements may be processed by a separate image processor for forming the image information before transmitting the image information to the processing unit 140. Regardless of where the image information is determined, the hardware and/or software configured to determine the image information should be construed as part of the image sensor 130.

The processing unit 140 may be configured to receive the recorded electrical signals and to process the recorded electrical signals to determine an electrical representation relating to an intracellular and/or extracellular action potential and/or an impedimetric electrical image of a cardiomyocyte. The processing unit 140 may receive a plurality of simultaneous recorded electrical signals applying to different cardiomyocytes on the substrate 110, such that a plurality of electrical representations for individual cardiomyocytes on the substrate 110 may be determined.

The determined electrical representation may provide a measure of intracellular action potential and/or extracellular action potential. This implies that the electrical representation may provide information relating to opening and closing of ion channels within and/or between cells which play a central role in activation of processes in and/or between cells. The extracellular action potential may be obtained as a consequence of determining a potential at a site of an electrode in the microelectrode array 112, as may be read out by the readout circuitry 120, whereas the intracellular action potential may in addition need electroporation in order to allow intracellular access for the electrodes in the microelectrode array 112.

The determined electrical representation may also or alternatively provide a spectroscopic electrical impedance measurement in the form of an electrical impedimetric image. The electrical impedimetric image may give information about cell viability, attachment and movement. This may be obtained as a consequence of determining an impedance between two closely arranged electrodes in the microelectrode array 112, as may be read out by the readout circuitry 120, wherein a plurality of pairs of closely arranged electrodes in the microelectrode array 112 allow obtaining of a two-dimensional image.

The processing unit 140 may further be configured to receive the image information, e.g. from a process or portion within the processing unit 140 or from a separate image processor, and to process the image information to determine a contractile representation relating to cellular deformation during contractility action of a group of cardiomyocytes. The determination of the contractile representation will be described in further detail below.

The processing unit 140 may extract a plurality of contractile representations based on dividing image information into a plurality of regions of interest and determining contractile information for each region of interest.

The processing unit 140 may further extract one or more parameters based on the electrical representation and the contractile representation, as will be further described below. The one or more parameters may provide characteristics of cardiomyocyte function which may be used for comparing between different measurements on cardiomyocytes, which may enable drawing conclusions on the cardiomyocyte function.

The processing unit 140 may be implemented in hardware, or as any combination of software and hardware. At least part of the functionality of the processing unit 140 may, for instance, be implemented as software being executed on a general-purpose computer. The device 100 may thus comprise one or more processing units, such as a central processing unit (CPU), which may execute the instructions of one or more computer programs in order to implement desired functionality.

The processing unit 140 may alternatively be implemented as firmware arranged e.g. in an embedded system, or as a specifically designed processing unit, such as an Application-Specific Integrated Circuit (ASIC) or a Field-Programmable Gate Array (FPGA).

The device 100 may further comprise a controller 142, which may be implemented as part of the processing unit 140 or which may be a separate unit, implemented in hardware, or as any combination of software and hardware as described above for the processing unit 140.

The controller 142 may provide signals for controlling functionality of the device 100. The controller 142 may also comprise an internal clock, which may be used in order to synchronize functionality of the device 100 and/or to determine a temporal relationship between recorded electrical signals and acquired image information.

The controller 142 may provide pacing of the cardiomyocytes by triggering stimulation signals to be provided to one or more cardiomyocytes. The controller 142 may further trigger recording of electrical signals and acquiring of image information in relation to the pacing of the cardiomyocytes.

The device 100 may comprise a housing 150 in which components of the device 100 may be arranged. Thus, a measurement position may be defined within the housing 150 and the readout circuitry 120 and the image sensor 130 may be arranged within the housing 150 in a predefined relation to the measurement position. In particular, arrangement of the image sensor 130 in the housing 150 may ensure that the image information is acquired with a well-defined relation between the image plane of the image sensor 130 and the measurement position in which the substrate 110 with the cardiomyocytes will be placed.

The light source 134 may be part of the device 100 and may be arranged in the housing 150, so as to provide a well-defined set-up between the light source 134, the measurement position and the image sensor 130. However, the light source 134 may be separately provided and an optical set-up including the light source 134 may be established by an end-user.

It should also be realized that the processing unit 140 need not necessarily be arranged in the housing 150. On the contrary, the processing unit 140 may be arranged in an external unit, and the device 100 may comprise a communication unit in the housing 150 for transmitting preprocessed or raw data to the external unit for processing. Communication with the external unit may be performed via a wired or wireless connection and may include communication over a computer network, such as the Internet, enabling the processing unit 140 to be arranged virtually anywhere, e.g. in "the cloud".

The device 100 may also be provided with a display. The device 100 may thus be arranged to display a visual representation of the electrical representation and the contractile representation, such as curve profiles as will be described in further detail below.

With display of curve profiles, an experienced user may draw conclusions based on displayed curve profiles, such that the device 100 need not necessarily extract parameters from the electrical representation and the contractile representation. However, display of the curve profiles may further be enhanced by also displaying values of one or more parameters extracted from the electrical representation and/or the contractile representation.

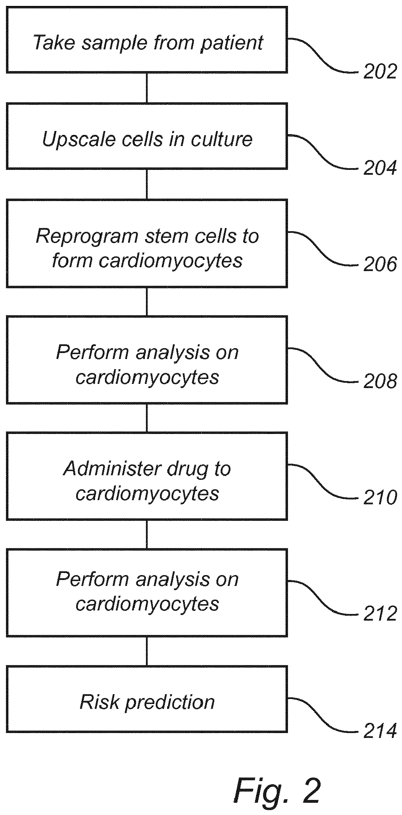

Referring now to Fig. 2, an overview of a method utilizing analysis of cardiomyocyte function will be described. The overview may provide a context in which the analysis is especially advantageous, but it should be realized that the analysis of cardiomyocyte function may be used in other contexts as well.

The analysis of cardiomyocyte function may be individualized such that the cardiomyocyte function of a specific patient may be determined. This may be used for cardiotoxicity assessment of a drug for a particular patient, such that a determination of which drug that would be suitable to use for the particular patient may be obtained.

First, a skin biopsy or a blood sample from the patient may be taken, 202. Thus, cells of the patient are acquired, which cells may be transferred back to stem cells and upscaled 204 in a cell culture to a large number of cells. The stem cells may be arranged on a substrate 110 provided with a microelectrode array 112 so as to prepare for desired measurements on the cells.

Then, stem cells may be reprogrammed 206 for forming of cardiomyocytes on the substrate 110 and the cardiomyocytes may further grow on the cell culturing surface of the substrate 110. Thus, a personalized sample of cardiomyocytes is formed on the substrate 110 enabling analysis of cardiomyocyte function of the particular patient.

The substrate 110 may be arranged in the measurement position of the device 100 and analysis 208 on the cardiomyocytes may be performed. Thus, an electrical representation and a contractile representation may be determined and, optionally, one or more parameters may be extracted.

During or in-between measurements, a drug may be administered 210 to the cardiomyocytes in order to allow predicting of adverse effects on electrical and/or contractile function of the cardiomyocytes.

After drug treatment, analysis 212 on the cardiomyocytes is again performed. The electrical and/or contractile representations before and after drug treatment may be compared in order to predict adverse effects.

Thanks to the measurements being non-invasive, the analysis before and after drug treatment may be performed on the same cells.

The processing unit 140 may perform comparison and provide output of comparison e.g. between one or more parameters extracted from the electrical and/or contractile representations.

Additionally or alternatively, results before and after drug treatment may be presented to a physician, such as a cardio-oncologist, who may perform a risk prediction 214 of administering the drug to the patient based on presented results. Thus, a suitable drug and/or a suitable drug concentration to be administered to the patient may be determined, e.g. by testing effects of a plurality of drugs in parallel and/or a plurality of dosages of drugs.

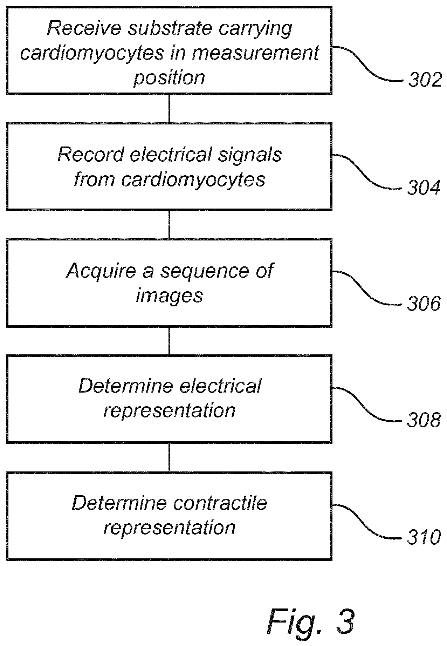

Referring now to Fig. 3, a method of analyzing cardiomyocyte function will be described.

The method comprises receiving 302 in a measurement position a substrate 110 carrying cardiomyocytes.

The method further comprises recording 304 electrical signals from the cardiomyocytes. The electrical signals may be acquired by the microelectrode array 112, which may be arranged in contact with the cardiomyocytes on the substrate 110. The electrical signals may further be transferred to readout circuitry 120 recording electrical signals received from the microelectrode array 112.

The method further comprises simultaneously with the recording 304, acquiring 306 a sequence of images. Each image may be acquired by detecting light wavefront information in reflective lensfree imaging and reconstructing the image of the cardiomyocytes based on the detected light wavefront information.

The method further comprises determining 308 an electrical representation relating to an intracellular and/or extracellular action potential, and/or an impedimetric electrical image of a cardiomyocyte based on the recorded electrical signals. Thus, information of electrical function of the cardiomyocyte is determined.

The method further comprises determining 310 a contractile representation relating to cellular deformation during contractility action of a group of cardiomyocytes based on the acquired sequence of images. Thus, information of contractile action of the cardiomyocytes is determined.

The electrical representation and the contractile representation may apply to a common period of time, which implies that electrical and contractile function of the cardiomyocytes may be simultaneously analyzed and may be determined for the same cells.

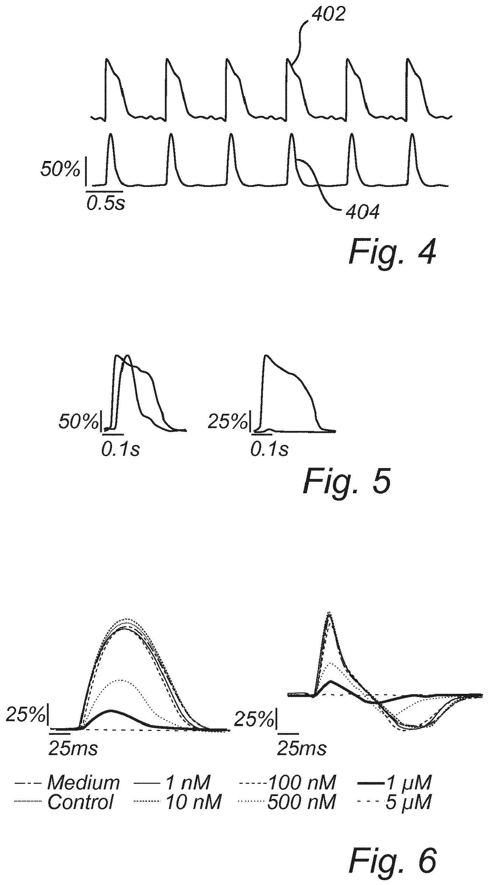

Referring now to Fig. 4, the electrical representation and the contractile representation and a process of determining the representations will be further described.

The microelectrode array 112 together with the readout circuitry 120 may record a time sequence corresponding to a variation of electrical potential locally in the cardiomyocytes on the cell culturing surface. This electrical potential may relate to an intracellular action potential and/or an extracellular action potential. The microelectrode array 112 may be set up to either detect a potential relating to an intracellular action potential or an extracellular action potential. Alternatively, one or more first electrodes of the microelectrode array 112 may be configured to detect a potential relating to an intracellular action potential, whereas one or more second electrodes, different from the first electrodes may be configured to detect a potential relating to an extracellular action potential.

Extracellular action potential may be determined based on an electrical potential that may be directly sensed by an electrode.

In order to detect an electrical potential relating to an intracellular action potential, intracellular access may be provided to electrodes of the microelectrode array by local membrane electroporation using subcellular electrodes. Duration of the intracellular access may be tuned by adapting the electroporation protocol. Use of electroporation for providing intracellular access is minimally invasive and may be used in consecutive recordings from the same cell over a long period of time.

As shown by the upper line in Fig. 4, a curve profile 402 may be formed based on the time sequence of the recorded electrical signal from an electrode in the microelectrode array 112, representing the time variation of the recorded potential. As illustrated in Fig. 4, the curve profile 402 representing intracellular action potential for a well-functioning cardiomyocyte may exhibit a very fast increased potential to build up an action potential to reach a maximum when the potential may no longer be increased. Then, transport of ions is reversed and the action potential is decreased again to reach a rested state. This process periodically repeats as illustrated in Fig. 4.

Alternatively or additionally, a spectroscopic electrical impedance measurement may be performed using the microelectrode array 112. An electrical impedimetric image may be formed as an electrical representation of the spectroscopic electrical measurement. This may be obtained as a consequence of determining an impedance between two closely arranged electrodes in the microelectrode array 112, as may be read out by the readout circuitry 120, wherein a plurality of pairs of closely arranged electrodes in the microelectrode array 112 allow obtaining of a two-dimensional image.

In the spectroscopic elelctrical impedance measurement, for a given frequency of an AC signal generated for pacing cardiomyocytes, the impedance of the cell is determined. The electrical impedance measurement may be performed for a range of different frequencies of the AC signal. The electrical impedance image may be acquired by sweeping a frequency range of the AC signal. For instance, the frequency may be swept between 1 Hz - 1 MHz. Each pair of electrodes in the plurality of pairs of electrodes may acquire electrical impedances for the frequencies in the frequency range.

According to an alternative, the electrical impedance image may alternatively be acquired using a fixed frequency of the AC signal. In such case, changes in the impedance at the frequency of the external field may be monitored over time.

The determined electrical impedimetric image may provide insight to how strongly the cell is attached to the electrode, how the cell membrane moves over time on the electrode, and thus, how drugs may influence such parameters.

As shown by the bottom line in Fig. 4, a curve profile 404 may be formed based on a sequence of acquired images. However, the acquired images may need to be processed in order to extract information that may allow representing time variation of contractile action of a group of cardiomyocytes as a curve profile 404.

A relative cellular deformation may be extracted from the images and may be used as a measure for forming the contractile representation as the curve profile 404. Also or alternatively, a rate of cellular deformation may be extracted and used as a measure for forming a contractile representation.

First, each image may be divided into a plurality of regions of interest. Each region of interest may be separately analyzed and the analysis may form a contractile representation for the group of cardiomyocytes within the region of interest.

A region of interest may for instance correspond to 64x64 pixels in the image. However, a size of the region of interest may depend on the optical set-up and resolution of the reflective lensfree imaging. Typically, the region of interest may correspond to an area on the substrate 110 comprising several cardiomyocytes, such as an area of approximately 0.12 mm², which may include 10-50 cardiomyocytes.

In order to determine contractile information in a region of interest of an image, the information in the region of interest may be compared to a reference. Thus, a difference in relation to the reference may be determined which may be used for determining a measure of the contractile action of the group of cardiomyocytes imaged in the region of interest.

Herein, the terms "image" and "frame" may be used interchangeably.

A reference frame may be determined based on one or more images in the region of interest, such that relative changes in the region of interest are determined. The reference frame may be determined in many different ways.

For instance, a single image is selected to form a predetermined reference frame. This is a very simple manner of selecting the reference frame. However, this may lead to drift in the determined relative cellular deformation and hence low quality of analysis.

An average reference frame may be formed. The average reference frame may be determined by setting pixel values of the average reference frame to be an average pixel value of the pixel in all frames in a sequence. However, for cells beating at a high frequency, this may lead to a distorted peak shape of the determined relative cellular deformation.

A moving reference frame may be formed. Thus, for each frame at a time instant t, a reference frame is calculated as an average reference frame based on the frames in an interval [*t-x*:*t*+*x*].

A reference frame to be used in analysis of a current frame may be selected based on a previous frame. Thus, the current frame at a time instant *t* may be compared to a frame taken *dt* seconds before at time instant *t-dt.* However, this implies that the reference frame is constantly changing. Determination of difference in relation to such changing reference may imply that an indication of a rate of relative cellular deformation is directly determined. However, such determination of an indication of a rate of relative cellular deformation is relatively sensitive to noise and provides a limited time resolution.

A reference frame may be determined based on a "relaxed" state of the group of cardiomyocytes. This may require determining when the group of cardiomyocytes are relaxed, but the relative cellular deformation determined based on a relaxed reference frame may provide a low sensitivity to noise and a clear peak shape of a curve profile 404 representing relative cellular deformation.

Determination of the relaxed reference frame may include determining time instants at which the cardiomyocytes are contracting. This may be performed by detecting maxima in pixel intensity variation between sequential frames. This process is a fast and easy way to initially determine if and when cells are beating.

The detecting of maxima in pixel intensity variation may include calculating a pixel intensity variation for all pixels in a region of interest as compared to a predetermined reference frame (see above). Then, an absolute value of pixel intensity variation for all pixels is determined. Further, the absolute value of the pixel intensity variation may be summed over all pixels in the region of interest so as to obtain a time varying sequence of pixel intensity variations. Then, peak maxima in the sequence may be detected.

Thus, when time instants of contraction have been determined in such manner, frames halfway between two peak maxima, i.e. between two contractions are extracted. These frames correspond to the cardiomyocytes being in a relaxed state. The frames selected in this manner may be averaged and used as a reference frame.

A measure of a relative cellular deformation of a frame may be determined by calculating motion vectors between the frame and the reference frame. The motion vectors may be determined by comparing local neighborhoods for each pixel with the reference and determining a translation between the local neighborhoods in a current frame and the reference frame. In this way, a two-dimensional displacement field between the current frame and the reference frame may be determined. For instance, the Farneback algorithm may be used.

The relative cellular deformation of a frame may then be determined by summing up all the motion vectors in the region of interest. In this way, a single value of the relative cellular deformation in the region of interest for a frame is determined. Then, a time-varying representation of the relative cellular deformation may be formed as a time sequence of the values of the relative cellular deformation.

The time-varying representation may be further smoothed and temporally interpolated in order to form the curve profile 404 as shown in Fig. 4.

A rate of relative cellular deformation may be formed by determining a first derivative of the curve profile 404 representing the relative cellular deformation.

Representations of the relative cellular deformation and the rate of relative cellular deformation may be determined for each region of interest.

The curve profiles 402 and 404 may be further analyzed in order to extract electrical and contractile parameters, which may be used in further analysis of the cardiomyocyte function.

A beat-to-beat duration may be determined based on either one of the curve profile 402 or 404. For instance, based on the relative cellular deformation, a beat-to-beat duration may be determined as a duration between consecutive starts of contraction. The start of contraction may be defined as a time point in which a smoothed second derivative of the curve profile 404 representing relative cellular deformation reaches a maximum value. Further, beating rates may be determined based on beat-to-beat durations.

Further, amplitudes of the curve profiles 402, 404 may be determined. Thus, amplitude may be determined as a peak value, i.e. the peak value of action potential or peak value of contraction. Also, an amplitude of a rate of relative cellular deformation may be determined as a difference between a positive and a negative peak.

Further, contraction durations may be determined as a duration during which the relative cellular deformation exceeds a threshold, e.g. 20% of a peak value of the contraction. Similarly, an action potential duration may be determined as a duration during which the electrical signal exceeds a threshold.

Further, an averaged action potential, averaged relative cellular deformation, and averaged rate of relative cellular deformation may be determined by overlapping curve profiles for single beats.

Also, by determining the electrical representation and the contractile representation at different parts of the substrate 110, propagation of an excitation wave may also be determined. The propagation of the excitation wave may be determined by comparing temporal relationships between action potentials or contractile action for different cardiomyocytes.

For instance, the temporal relationship between regions of interest may be determined. In each region of interest, a time point at which contraction is initiated may be determined and compared to a time point in a region of interest wherein contraction is first detected.

By determining an average delay for all regions of interest, the propagation velocity may further be calculated by averaging the speed at which the excitation wave propagates from the region of interest wherein contraction is first detected to the region of interest farthest away within a field of view of the image sensor 130.

Referring to Figs 5-6, an example of a comparison of the cardiomyocyte function before and after administering of a drug is discussed. In the left part of Fig. 5, an electrical representation and a contractile representation are shown before administering of a drug. In the right part of Fig. 5, an electrical representation and a contractile representation are shown after administering of drug (blebbistatin). As shown in Fig. 5, the electrical function is not affected. However, the drug has severe adverse effects on the contractile function of the cardiomyocytes.

In Fig. 6, relative cellular deformation (left part of Fig. 6) and a rate of relative cellular deformation (right part of Fig. 6) is shown for different drug concentrations being administered. Hence, as illustrated in Fig. 6, the effect of different concentrations may also be studied by the cardiomyocyte function analysis provided herein.

In the above the inventive concept has mainly been described with reference to a limited number of examples. However, as is readily appreciated by a person skilled in the art, other examples than the ones disclosed above are equally possible within the scope of the inventive concept, as defined by the appended claims.

For instance, it should be realized that the contractile representation may be determined in many different ways using different types of image processing and the particular types of image processing described herein should not be considered as limiting the scope of protection.

## Claims

1. A method for analysis of cardiomyocyte function, said method comprising:
receiving (302) in a measurement position a substrate (110) carrying cardiomyocytes on a cell culturing surface, wherein the substrate (112) comprises a microelectrode array (112) in contact with the cardiomyocytes;
recording (304) electrical signals from the cardiomyocytes, said electrical signals being acquired by the microelectrode array (112);
simultaneously with said recording, acquiring (306) a sequence of images of the cardiomyocytes on the cell culturing surface, wherein each image in the sequence of images is acquired by detecting light wavefront information of reflected light, the light wavefront information forming an interference pattern between diffracted light from the cardiomyocytes and undiffracted light, and digitally calculating image information based on the detected light wavefront information;
determining (308) an electrical representation relating to an intracellular and/or extracellular action potential, and/or an impedimetric electrical image of a cardiomyocyte based on the recorded electrical signals,
determining (310) a contractile representation relating to cellular deformation during contractility action of a group of cardiomyocytes based on the acquired sequence of images, wherein the electrical representation and the contractile representation apply to a common period of time.

2. The method according to claim 1, further comprising administering (210) a drug to the cardiomyocytes and comparing the electrical representation and the contractile representation acquired before administering of the drug with the electrical representation and the contractile representation acquired after administering of the drug.

3. The method according to claim 2, further comprising, based on said comparing, determining whether the drug has electrical adverse effects on the cardiomyocytes and determining whether the drug has contractile adverse effects on the cardiomyocytes.

4. The method according to any one of the preceding claims, further comprising sending an electrical signal to one or more cardiomyocytes for pacing the cardiomyocytes.

5. The method according to any one of the preceding claims, further comprising synchronizing the recorded electrical signals with the acquired sequence of images.

6. The method according to any one of the preceding claims, wherein the images in the acquired sequence of images are divided into a plurality of regions of interest, wherein each region of interest represents a group of cardiomyocytes.

7. The method according to claim 6, wherein determining of a contractile representation comprises, for each image, determining a vector sum representing a displacement field in relation to a common reference, and forming a curve profile (404) representing relative cellular deformation based on a sequence of the determined vector sums.

8. The method according to claim 7, wherein the determining of a contractile representation further comprises forming a curve profile representing a rate of relative cellular deformation based on a first derivative of the curve profile (404) representing relative cellular deformation.

9. The method according to claim 7 or 8, further comprising determining an average curve profile based on a plurality of beat curve profiles for determining the curve profile representing relative cellular deformation and the curve profile representing the rate of relative cellular deformation.

10. The method according to claim 8 or 9, further comprising extracting a beating rate from the curve profile (404) representing relative cellular deformation and the curve profile representing the rate of relative cellular deformation.

11. The method according to any one of claims 8-10, further comprising extracting a contraction duration based on the curve profile (404) representing relative cellular deformation by determining a period of time for which the curve profile (404) exceeds a threshold related to a peak value of the curve profile (404).

12. The method according to any one of claims 8-11, further comprising extracting parameters based on a shape of the curve profile (404) representing relative cellular deformation and the curve profile representing the rate of relative cellular deformation.

13. The method according to any one of claims 6-12, further comprising determining, for each region of interest, a point in time of a phase of contraction, and determining a propagation velocity of an excitation wave based on difference in the point in time for different regions of interest.

14. The method according to any one of the preceding claims, further comprising extracting parameters based on a curve profile (402) representing an intracellular and/or extracellular action potential, and/or an impedimetric electrical image.

15. A device (100) for analysis of cardiomyocyte function, said device (100) comprising:
readout circuitry (120) for reading out electrical signals from a substrate (110) comprising a microelectrode array (112) in contact with cardiomyocytes on a cell culturing surface of the substrate (110);
an image sensor (130) arranged such that the cell culturing surface of the substrate (110) is facing the image sensor (130) when the substrate (110) is arranged to allow the readout circuitry (120) to read out the electrical signals, the image sensor (130) being configured to, simultaneously with reading out of electrical signals by the readout circuitry (120), acquiring a sequence of images of the cardiomyocytes on the cell culturing surface, wherein each image in the sequence of images is acquired by detecting light wavefront information of reflected light, the light wavefront information forming an interference pattern between diffracted light from the cardiomyocytes and undiffracted light, and digitally calculating image information based on the detected light wavefront information;
a processing unit (140) configured to receive the electrical signals from the readout circuitry (120) and image information from the image sensor (130), said processing unit (140) being further configured to determine an electrical representation relating to an intracellular and/or extracellular action potential, and/or an impedimetric electrical image of a cardiomyocyte based on the recorded electrical signals, and to determine a contractile representation relating to cellular deformation during contractility action of a group of cardiomyocytes based on the acquired sequence of images, wherein the electrical representation and the contractile representation apply to a common period of time.
